# EUROPÄISCHE PATENTSCHRIFT

(11) **EP 3 993 689 B1**
(45) Veröffentlichungstag und Bekanntmachung des Hinweises auf die Patenterteilung: **07.06.2023**
(21) Anmeldenummer: 20797667.1
(22) Anmeldetag: 14.10.2020
(51) Int. Cl.: A61B 1/00

(54) **KONTAMINATIONSSCHUTZVORRICHTUNG FÜR ENDOSKOPE MIT SEITWÄRTSOPTIK**
ANTI-CONTAMINATION DEVICE FOR ENDOSCOPES WITH SIDEWAYS OPTICS
DISPOSITIF ANTI-CONTAMINATION POUR ENDOSCOPES À OPTIQUE LATÉRALE

(30) Priorität: 16.10.2019 DE 102019127888
(43) Veröffentlichungstag der Anmeldung: 11.05.2022
(73) Patentinhaber: es endomed solutions GmbH, 84051 Essenbach (DE)
(72) Erfinder: KRESS, Jürgen, 84051 Essenbach (DE)
(74) Vertreter: Pöhner, Wilfried Anton
(86) Internationale Anmeldenummer: PCT/DE2020/100889
(87) Internationale Veröffentlichungsnummer: WO 2021/073692

(56) Entgegenhaltungen:
- EP-A2- 1 029 414
- EP-B1- 1 029 414
- WO-A1-2018/134666
- WO-A1-2018/134668

## Beschreibung

Es gibt in der Gastroenterologie neben den klassischen flexiblen Endoskopen, bei denen die Optik sowie die Öffnungen für Biopsie-, Absaug- und eventuelle weitere Kanäle für Luft und Wasserinsufflation im distalen Ende des Endoskopschafts frontseitig untergebracht ist auch anders konstruierte Endoskope.

Eine dieser Varianten, ein sogenanntes Endoskopisch Retrograde Cholangio-Pankreatikographie oder ERCP-Endoskop, wird für die Untersuchung und Behandlung der Gallenwege verwendet. Dabei wird ein kleiner Schlauch durch den Arbeitskanal des Endoskops geschoben und nach Verlassen der distalen Öffnung weiter in den Gallengang für die Durchführung der entsprechenden Maßnahmen. Da der Gallengang im Duodenum mündet, das sehr beengte räumliche Verhältnisse aufweist, kann die Endoskopspitze nicht wie notwendig auf den ca. im 90 (oder höheren) Gradwinkel eintreffenden Gallengang gerichtet werden. Aus diesem Grund haben diese Geräte eine Seitwärtsoptik am distalen Ende. Zudem verfügen sie über eine Mechanik an dieser Stelle, die es gestattet, den eingeführten Schlauch bzw das Instrument um 90 Grad zum Endoskop abzuwinkeln. Dies geschieht durch einen mechanisch zu bedienenden Hebel, der sich aufrichten lässt und eine leicht gekrümmte Gleitfläche aufweist, auf welcher ein durch den Biopsiekanal im Schaft des Endoskops geschobenes Instrument abgleitet und abhängig von der Stellung des Hebels mehr oder weniger stark umgebogen wird. Es ist dies der ALBARRAN-Hebel (benannt nach einem spanischen Arzt).

Diese Mechanik, Albarran-Hebel, Seilzug mit zuführendem Schacht, Gelenke, Exkavationen ist vollständig dem Kontakt von Körperflüssigkeiten bei der Untersuchung ausgesetzt. Wegen der vielen Ecken und der schlechten Zugänglichkeit bei der Reinigung nach der Untersuchung besteht hier ein großes Risiko einer unvollständigen Desinfektion.

Die vorliegende Erfindung löst daher das Problem der Infizierung der Endoskope und insbesondere der zerklüfteten Mechanik am distalen Geräteende.

Als Lösung wird ein Endoskop mit Kontaminationsschutzvorrichtung gemäß Schutzanspruch 1 vorgeschlagen. Dieses ist gekennzeichnet durch eine keimdichte Hülle, welche über den Gerätschaft gebracht wird und an der Stelle der Seitwärtsoptik eine transparente Fläche aufweist. Dieses kann in der Art eines Fensters ein Ausschnitt in der Seite einer ansonsten aus intransparentem Material bestehenden Hülle sein, wobei der Ausschnitt mit einem keimdicht mit der restlichen Hülle verbundenen transparenten Material, etwa einer Kunststofffolie, verschlossen ist. In alternativen Ausführungen ist das distale Ende der Hülle, welches über dem distalen Ende des Endoskopschafts zu liegen kommt, und diesen enganliegend umhüllt, jedoch zumindest in einem etwa zylinderförmigen Bereich aus einem transparenten Material gefertigt.

Die Hülle kann in manchen Ausführungsformen an ihrem distalen Ende mit einer das distale Ende des Endoskopschafts überdeckenden Kappe keimdicht verbunden sein. Abnehmbare Kappen aus Kunststoff zur Abdeckung der Mechanik sind im Stand der Technik zwar bekannt, da sie jedoch Öffnungen und Durchbrechungen aufweisen, damit sie von Biopsieinstrumenten sowie Absaug-, Luft und/oder Wasserkanälen durchbrochen werden können, schützen sie das distale Ende des Endoskops und die dortige Mechanik und Optik aber nicht vor Infizierung.

Es wird von vorliegender Erfindung für manche Ausführungsformen vorgeschlagen, die mit der Hülle keimdicht verbundene Kappe in ihrer Form den bekannten, abziehbaren Plastikkappen nachzuempfinden, hierbei aber die offene Seite der Kappe, also den Ausschnitt in der Kappe, der die Optik und Beleuchtung freilässt und von einem aus dem Arbeitskanal des Endoskops heraustretenden Instrument durchgriffen wird, mit einer Abdeckung zu versehen. Diese ist keimdicht mit den Rändern des Ausschnittes verbunden und damit geeignet, die offenliegende, sichtbare Mechanik, d.h. den Albarran-Hebel, keimdicht abzudecken.

Diese Abdeckung hat die Form eines dünnwandigen Kunststoffformteils welches die Öffnungen in der Kappe vollständig überdeckt. Sie wird von einem dünnwandigen Schlauch durchbrochen, der den Arbeitskanal ausfüllt und am Durchbruch durch die Abdeckung keimdicht mit ihr verbunden ist. Dies kann bereits werkseitig geschehen, d.h. die Kappe wird mit bereits keimdicht verbundener Abdeckung ausgeliefert, oder die Verbindung wird erst vor Ort im Zuge der Vorbereitung des Endoskops auf eine Untersuchung/Behandlung hergestellt. Insbesondere wird bei letzterem Vorgehen erst die Schafthülle über den Endoskopschaft gestreift bzw. kondomartig abgerollt, dann die Kappe aufgesetzt, wobei auf die korrekte Platzierung des Ausschnitts oberhalb der Optik und/oder einer Instrumentenkanalaustrittsöffnung zu achten ist, und mit einem offenen Ende der Hülle verbunden. Anschließend oder auch zuvor wird eine den Instrumentenkanal, auch Arbeitskanal genannt, auskleidender Arbeitskanalschlauch eingeführt bis er ein wenig aus der Mündung des Arbeitskanals in der Endoskopspitze herausragt. Nach diesen Schritten wird die Abdeckung auf den offenen Ausschnitt der Kappe aufgesetzt, wobei der Arbeitskanalschlauch die Abdeckung perforiert, und keimdicht sowohl mit der Kappe als auch dem Arbeitskanalschlauch verbunden.

Um eine gleichmäßige, leicht abdichtbare Perforation zu gewährleisten, wird vorgeschlagen, die Abdeckung herstellungsseitig mit einer Öffnung zu versehen, welche in Umriss und Größe dem Arbeitskanalschlauch angepasst ist. Um eine flächige Anlage der Abdeckung an den Arbeitskanalschlauch im Bereich der Perforation zu gewährleisten wird die Öffnung bevorzugt mit einem umlaufenden Kragen versehen.

Als Maßnahmen, um eine keimdichte Verbindung herzustellen kommen im Rahmen der vorliegenden Erfindung insbesondere Verschweißen oder Verkleben zum Einsatz. Hierbei ist verschweißen bei einer werkseitigen Vorbereitung des erfindungsgemäßen Kontaminationsschutzes in einem benutzungsfertigen Zustand bevorzugt, wohingegen bei der oben beschriebenen Vor-Ort Vorbereitung verkleben die Einfachere Verbindungsmethode darstellt, da sie ohne Spezialwerkzeug auskommt.

Unterhalb bzw. innerhalb der Abdeckung kann der Albarran-Hebel bewegt werden. Er bewegt innerhalb der Abdeckung den diese perforierenden Schlauch und das darin laufende Diagnose/Therapie-Gerät. Der Schutz besteht also aus einer Schafthülle, einer distalen Kappe, einer Abdeckung der Mechanik, einem Schlauch zur Arbeitskanalauskleidung und einem Wasser/Luftzufuhr-Schlauchsystem.

Bei der Bewegung/Betätigung des Albarran-Hebels ergibt sich eine mechanische Belastung der distalen Schafthülle, insbesondere wenn diese wie vorliegend vorgeschlagen als Kappe ausgeführt ist. Für die Abdeckung ist ein Material mit ausreichender Elastizität zu wählen, so dass die Bewegung des Albarran-Hebels nicht beeinträchtigt wird bzw. die Abdeckung, die Kappe oder die Verbindung der beiden auch bei maximalem Ausschlag des Albaran-Hebels nicht reißen.

Eine weitere Ausführungsform des Kontaminationsschutzes vorliegender Erfindung für ERCP-Endoskope umfasst eine Kappe, welche ebenfalls ähnlich den bereits bekannten Einmalgebrauchskappen gestaltet ist, jedoch die Schaftspitze, also die gesamte Optik und Mechanik (Albarran-Hebel und Ansteuerung) des Endoskops, vollständig abdeckt. Eine zusätzliche separate Abdeckung wird somit nicht benötigt. Damit die Optik nebst Beleuchtung auch bei einer solchen Ausgestaltung der Kappe verwendbar sind, ist diese Variante der Kappe mit einem hochtransparenten Fenster oberhalb der Endoskopoptik versehen.

Diese Variante der Kappe verfügt über einen *längslaufenden Schlitz* in dem sich der Schlauch der Arbeitskanalauskleidung durch den Albarranhebel in Längsrichtung und damit mit Änderung seines Austrittswinkels bewegen lässt. Ein faltenbalgartiger und/oder elastischer Folienbereich befindet sich zwischen Schlauch und Kappe und füllt den Kappenschlitz dicht aus. Somit ist die Mechanik auch bei dieser Ausführungsform vollständig abgedeckt und keimdicht geschützt. Innerhalb des an die Kappe anschließenden Folienschlauches zur Schaftabdeckung verläuft auch der(die) Luft/Wasserkanal(-kanäle).

Unter der distalen Kappe und zwischen den Kleinteilen der Mechanik liegt eine hohes Verschmutzungs- und Keimpotential. Deshalb wird neuerdings gefordert zumindest Einmalkappen zu verwenden was bereits umgesetzt ist. Diese Lösung behebt aber nur das Problem der verbleibenden Verschmutzung innerhalb der Kappe, keineswegs das der Verschmutzung der Mechanik. Das ist erst durch vorliegende Erfindung vorteilhaft ermöglicht.

Im Folgenden sollen weitere vorteilhafte Weiterbildungen der Erfindung, die einzeln oder in Kombination realisierbar sind, sofern sie sich nicht gegenseitig offensichtlich ausschließen, vorgestellt werden.

Das erfindungsgemäße Endoskop mit Kontaminationsschutz hat in bevorzugten Ausführungsformen einen Arbeitskanal für Instrumente und in der Schaftspitze eine Mechanik, um den Austrittswinkel eines aus dem Arbeitskanal herausgeführten Instruments zu beeinflussen. Dies ist insbesondere ein sogenannter Albarran-Hebel mit der nötigen Ansteuerung über Bowdenzüge.

In weiteren bevorzugten Ausführungsformen hat das erfindungsgemäße Endoskop einen oder mehrere Schläuche für eine Luft- und/oder Wasserinsufflation. Dieser Schlauch kann insbesondere ein kombinierter Luft/Wasserschlauch oder aber zwei getrennte Schläuche für Luft und Wasser sein. Die Schläuche befinden sich bevorzugt innerhalb der Hülle, und sind im Bereich des distalen Endes der Hülle mit einem offenen Ende des Schlauches mit dieser verbunden.

Der Schlauch mündet hierbei bevorzugt in einer gegenüber einer lokalen Längsrichtung des Schaftes um einen Winkel von 30 - 90 Grad geneigten Fläche des distalen Hüllenendes, insbesondere wenn dieses als eine Kappe ausgestaltet ist. Besonders bevorzugt ist diese Fläche senkrecht zur Längsrichtung des Schaftes ausgerichtet.

Die Mündung des dabei derart geformt und gerichtet, dass ein aus ihr austretender Wasserstrahl zunächst auf die Optik und die Beleuchtung, bzw. den diese abdeckenden Bereich der Abdeckung der Kappe (erste Ausführungsform der Kappe) oder das diese abdeckende Fenster der Kappe (zweite Ausführungsform der Kappe) auftrifft, um diese zur reinigen. Darüber hinaus ist es jedoch auch bevorzugt, dass der Strahl auch nach seiner Berührung mit der Kappe als gebündelter Wasserstrahl (Jet oder Wasserjet) weiterströmt. Dies wird insbesondere durch einen möglichst flachen, streifenden Auftreffwinkel des Strahls auf der Kappe oberhalb der Optik erreicht.

Der Schlauch oder die Schläuche haben weiterhin bevorzugt an ihrem dem distalen Ende gegenüberliegenden kranialen Ende ein integriertes Ventil, welches so geformt ist, dass es leicht in eine vorhandene Ventilaufnahme des erfindungsgemäßen Endoskops gesteckt werden kann.

Die Hülle des Kontaminationsschutzes vorliegender Erfindung ist im Allgemeinen ein oder mehrlagig. In besonders bevorzugten Ausführungen ist sie insbesondere doppellagig.

Weitere Merkmale, Eigenschaften und Vorteile der Erfindung ergeben sich aus dem im Folgenden anhand der Figuren vorgestellten und erläuterten bevorzugten Ausführungsbeispiel. Dies dient nur der Illustration und soll die vorliegende Erfindung in keiner Weise einschränken.

Es zeigen
- Figur 1:: In vier Teilfiguren, perspektivische Ansichten des distalen Endes eines Endoskops mit Seitwärtsoptik, dessen Schaft mit einer Schutzhülle der erfindungsgemäßen Kontaminationsschutzvorrichtung bedeckt ist.
- Figur 2A:: In vier Teilfiguren, perspektivische Ansichten einer Kappe der erfindungsgemäßen Kontaminationsschutzvorrichtung gemäß einer ersten Ausführungsform.
- Figur 2B:: In vier Teilfiguren, perspektivische Ansichten des distalen Endes eines Endoskops mit Seitwärtsoptik welches mit der Kappe aus Figur 2A bedeckt ist.
- Figur 2C:: wie Figur 2B, wobei hier jedoch die Mechanik des Endoskops gestrichelt angedeutet ist.
- Figur 3A:: In vier Teilfiguren, perspektivische Ansichten des distalen Endes eines Endoskops mit der Kappe gemäß Fig. 2A, Abdeckung und einem herausgeführten Instrumentenkanalschlauch.
- Figur 3B:: wie Fig. 3A, wobei der Verlauf des Schlauches im Schaftinneren gestrichelt kenntlich gemacht ist.
- Figur 3C:: In vier Teilfiguren, weitere Ansichten des distalen Schaftendes mit herausgeführtem Instrumentenschlauch, wobei die Schaftspitze im Inneren der Kappe gestrichelt kenntlich gemacht ist.
- Figur 4A:: In vier Teilfiguren, perspektivische Ansichten der Abdeckung des offenen Ausschnittes der ersten Ausführungsform der Kappe und ihre Verbindung zu einem Instrumentenkanalschlauch, aber ohne Schaft oder Hülle.
- Figur 4B:: In vier Teilfiguren, weitere Ansichten der Abdeckung ihrer Verbindung zu einem Instrumentenkanalschlauch.
- Figur 4C:: In vier Teilfiguren, perspektivische Ansichten der Abdeckung aus den Figuren 4A und 4B und deren Anschluss an einen Absaugschlauch.
- Figur 5A:: In vier Teilfiguren, perspektivische Ansichten von Komponenten des Kontaminationsschutzes mit einer ersten Variante der Kappe gemäß der ersten Ausführungsform.
- Figur 5B:: In vier Teilfiguren, perspektivische Ansichten von Komponenten des Kontaminationsschutzes mit einer zweiten Variante der Kappe gemäß der ersten Ausführungsform.
- Figur 6:: In zwei Teilfiguren, Ansichten einer Kappe eines erfindungsgemäßen Kontaminationsschutzes gemäß einer zweiten bevorzugten Ausführungsform.

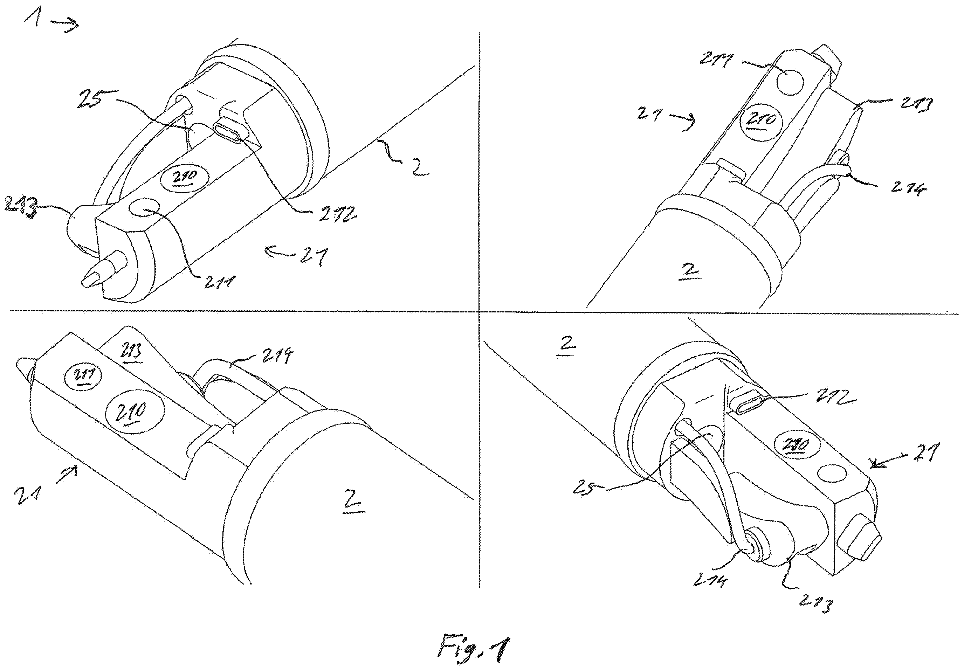

In **Figur 1** ist in vier Teilfiguren die Schaftspitze eines Endoskops mit Seitwärtsoptik dargestellt.

Zu sehen sind jeweils die Spitze 21 des Schaftes 2, welcher hier, wie üblich einen runden Querschnitt aufweist, und die in der Spitze 21 angeordnete seitwärts, d.h. senkrecht zur Längsrichtung des Schaftes 2 gerichtete Optik 210 mit zugeordneter, benachbarter Beleuchtung 211 welche ein Leuchtmittel (nicht dargestellt) in Form einer LED oder einer optischen Faser, durch welche externes Licht eingestrahlt wird, umfasst. Weiterhin ist die Mechanik zu erkennen, welche einen Albarranhebel 213 und einen zu dessen Bewegung nötigen Bowdenzug 214 umfasst. Ebenfalls zu sehen ist die Mündung 25 eines Instrumentenkanals und die Mündung 212 eines Luft und/oder Wasserkanals im Inneren des Schaftes 2.

Durch Optik und insbesondere die Mechanik hat die Schaftspitze eine sehr zerklüftete Geometrie, was eine vollständige Kontamination nach einer Untersuchung ohne den erfindungsgemäßen Kontaminationsschutz sehr aufwendig, langwierig und riskant macht.

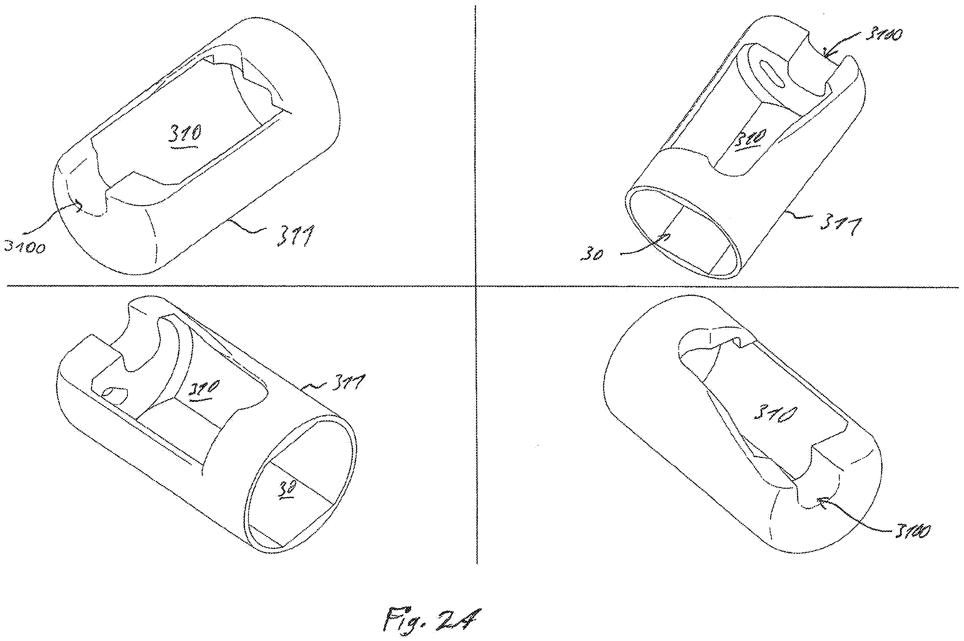

In den **Figuren 2A** - **C** ist eine erste Ausführungsform der Kappe eines erfindungsgemäßen Kontaminationsschutzes und ihre Verwendung in Verbindung auf der Schaftspitze gezeigt.

**Figur 2A** zeigt die Kappe 311 welche über ein offenes Ende 30 zum Aufstecken auf die Schaftspitze eines Endoskops und einen Ausschnitt 310 für die Optik, Beleuchtung und ein Instrument verfügt.

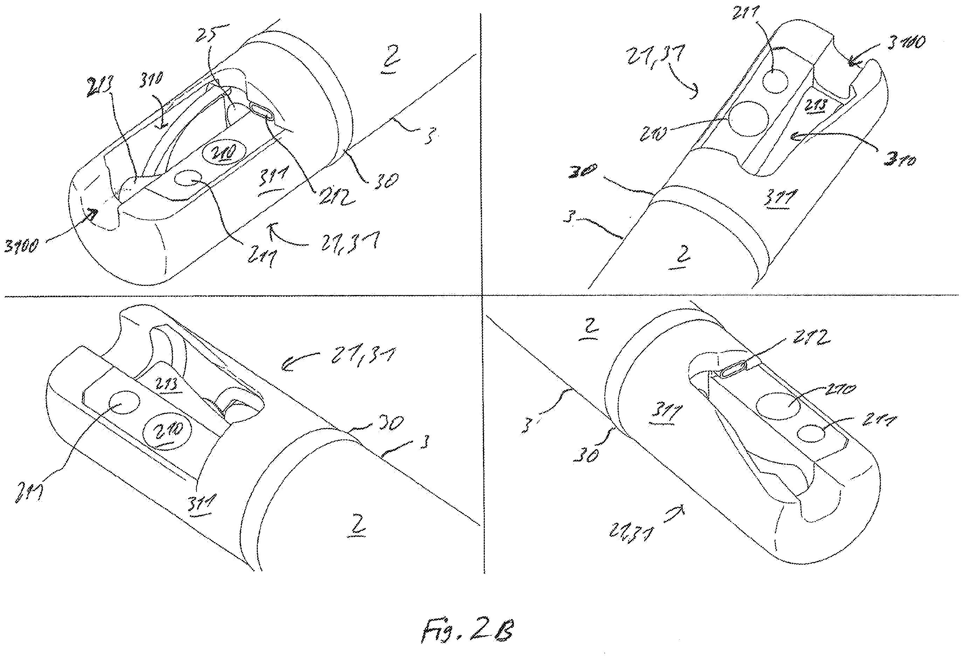

In **Figur 2B** ist die Kappe 311 auf die Schaftspitze 21 gesteckt gezeigt, wobei sie im Bereich 30 mit der den Schaft 2 eng anliegend überdeckenden Hülle 3 keimdicht verbunden, insbesondere verschweißt, ist. Aussparung 3100 des Ausschnittes 310 erlaubt hierbei ein Instrument auch dann ohne Beschädigung der Kappe aus der Endoskopspitze 21 herauszuführen, wenn der Albaranhebel 213 nur eine minimale Ablenkung vorgibt.

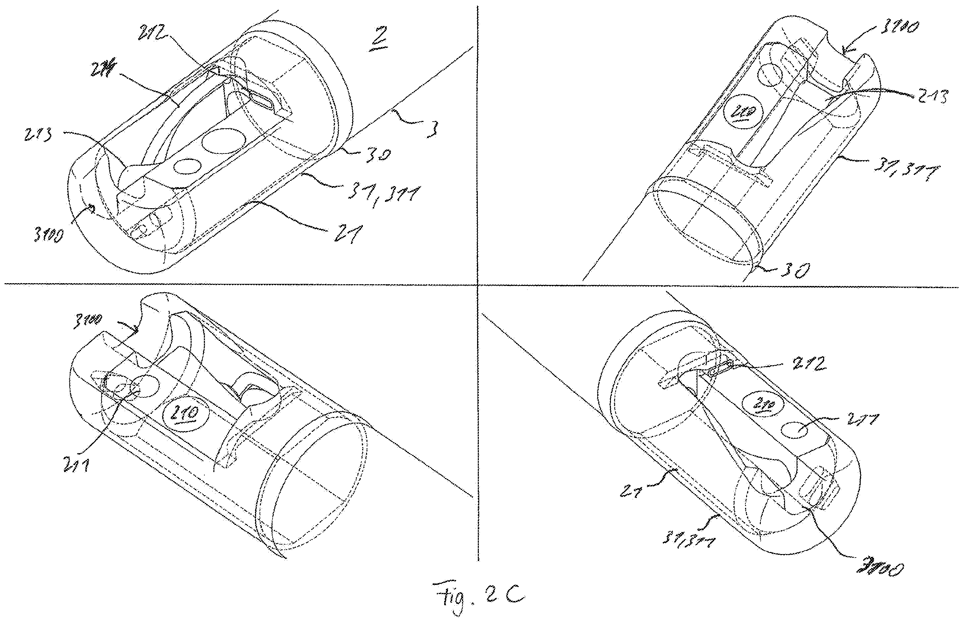

In **Figur 2C** sind zusätzlich zu Figur 2B noch gestrichelt die Schaftspitze 21 im Inneren der die distale Ende 31 der Hülle 3 bildenden Kappe 311 dargestellt.

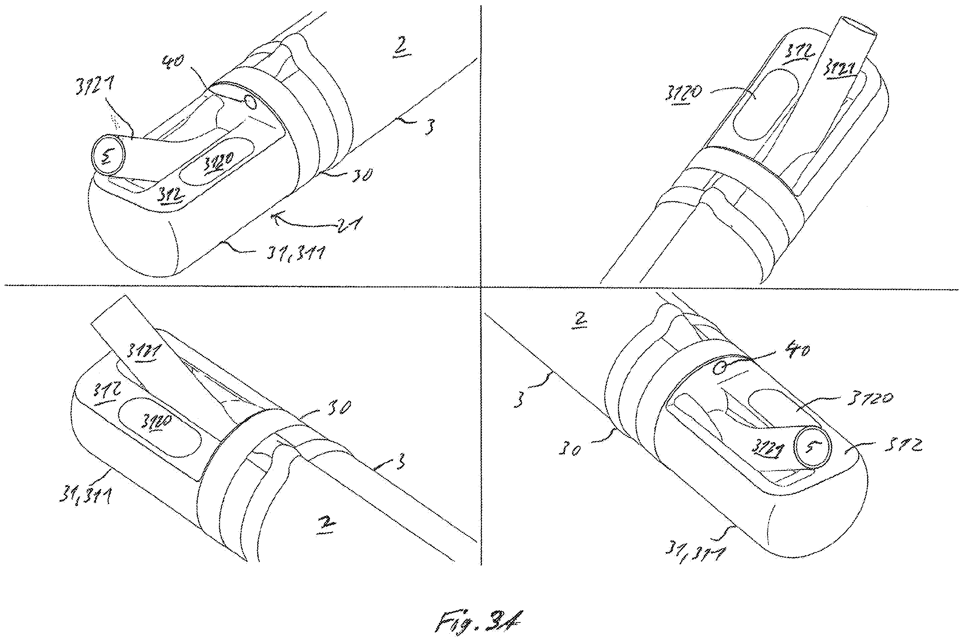

Die **Figuren 3A** - **C** zeigen die Kappe gemäß der ersten Ausführungsform aus Figur 2A mit einer Abdeckung zum keimdichten Verschluss offenen Ausschnitts der Kappe in Verbindung mit einem aus der Schaftspitze herausgeführten Instrumentenschlauch des erfindungsgemäßen Kontaminationsschutzes.

In **Figur 3A** sind vier unterschiedliche perspektivische Ansichten der Schaftspitze 21 mit aufgesteckter, das distale Ende 31 der Hülle 3 bildender Kappe 311. Ein, hier nicht sichtbarer, Ausschnitt der Kappe 311 ist durch die Abdeckung 312 vollständig keimdicht verschlossen. Abdeckung 312 wird durch den Instrumentenschlauch 5 durchdrungen und ist mit diesem entlang der Berührungsfläche des schlauchstutzenartigen Kragens 3121 der Abdeckung 312 flächig und keimdicht verbunden. Die Abdeckung 312 hat ein transparentes Fenster 3120 oberhalb der Optik und Beleuchtung (hier nicht sichtbar).

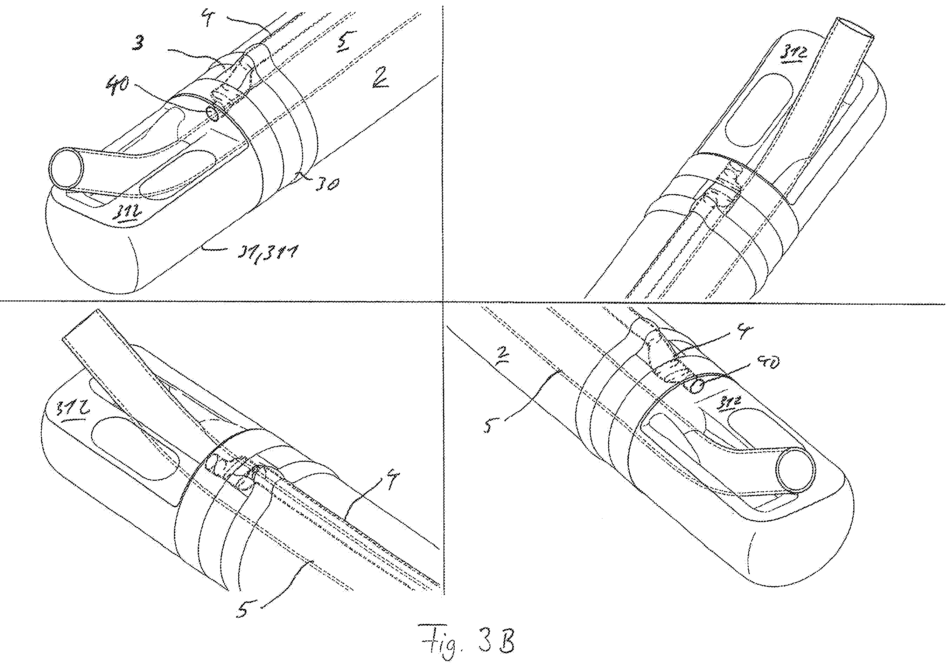

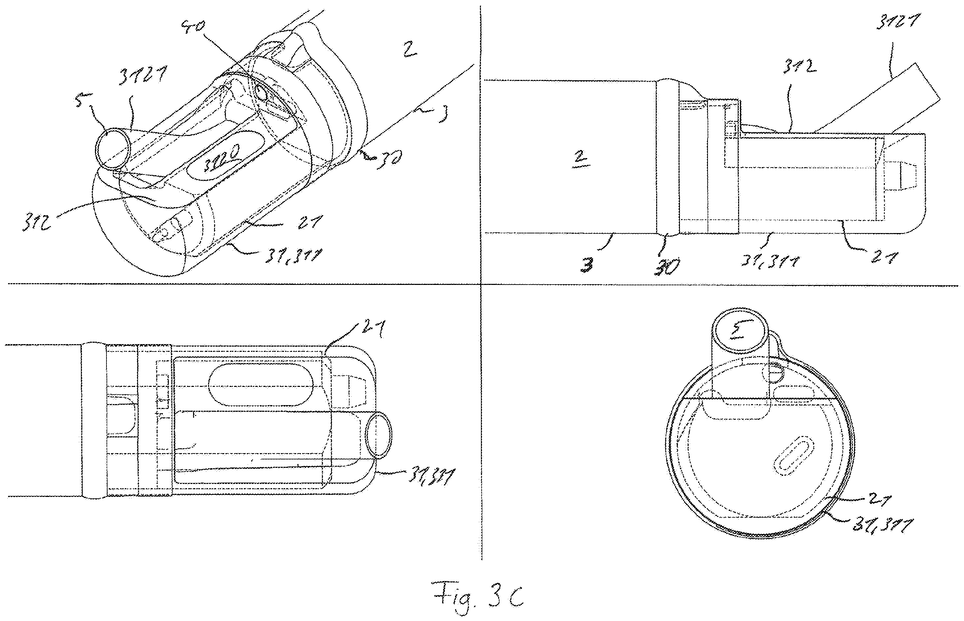

Die **Figuren 3B** und **3C** zeigen ähnliche perspektivische Ansichten wie Figur 3A, wobei jedoch das Innenleben des Schaftes 2 bzw. der Spitze 21 gestrichelt angedeutet sind. In **Figur 3B** ist so der Verlauf des Instrumentenkanalschlauches 5 im Inneren des Schaftes 2 zu erkennen, sowie der Verlauf eines Absaugschlauches 4 im Inneren der Hülle 3, welche den Schaft 2 eng anliegend überdeckt.

In **Figur 3C** ist die Schaftspitze 21 im Inneren von Kappe 311 gestrichelt angedeutet.

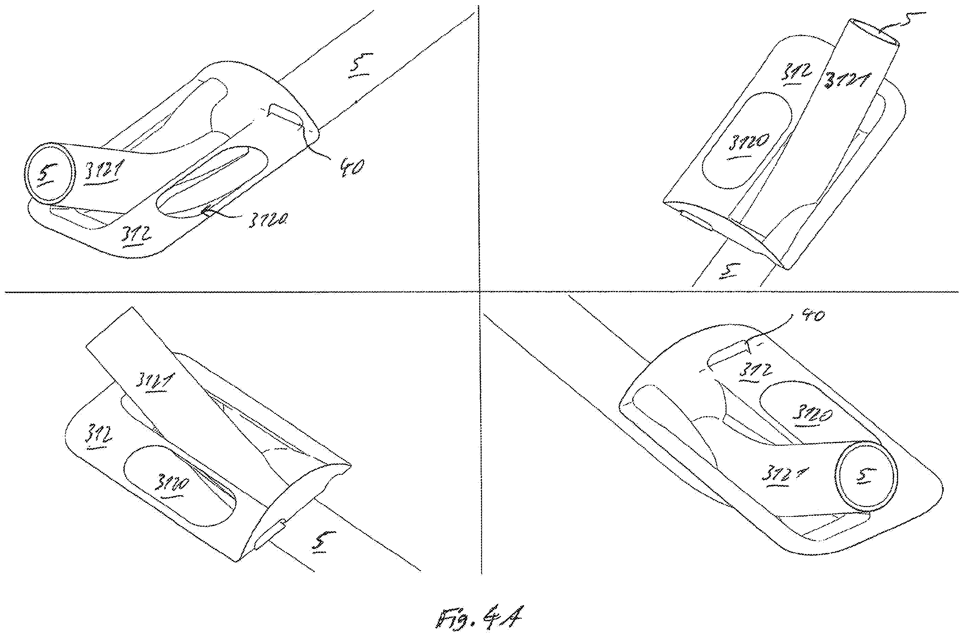

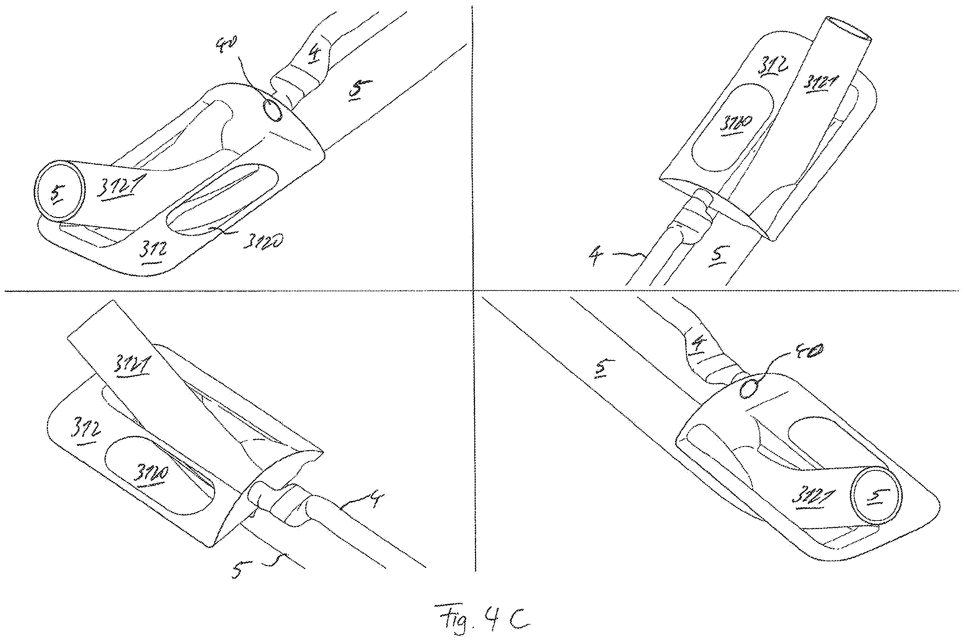

Die **Figuren 4A** - **4C** zeigen die Abdeckung 312 mit dem angeschlossenen Instrumentenschlauch 5 und Absaugschlauch 4 (Fig. 4C) unabhängig von der Schaftspitze oder der Kappe. Die Abdeckung 312 ist entlang des flächig das die Abdeckung 312 durchdringende offene Ende des Schlauches umschließenden Kragen 3121 mit dem Schlauch keimdicht verbunden. Sie weist wie hier dargestellt im Durchtrittsbereich des Schlauches 5 eine Vertiefung, also eine nach innen gewölbte Oberseite auf. Dies ist fakultativ und eine vollständig glatte Oberseite mit einer entsprechenden Verkürzung des Kragens 3121 wäre ebenfalls möglich. In **Figur 4C** ist zusätzlich noch ein an seiner Mündung 40 keimdicht an die Abdeckung angeschlossener Absaugschlauch 4 dargestellt.

Es wird vorliegend vorgeschlagen, in manchen Ausführungsformen der Erfindung Kappenabdeckung und Arbeitsschlauch sowie ggf. Absaugschlauch wie hier in den Figuren 4A-4C dargestellt als eine vorverbundene Einheit zu liefern, so dass der Instrumentenschlauch nach dem Aufsetzen der Kappe gemäß der ersten Ausführungsform nach Fig. 2A von der Endoskopspitze her in den Arbeits- bzw. Instrumentenkanal des Schaftes eingeführt wird, bis die Abdeckung auf den Rändern des Ausschnittes in der Kappe aufliegt. Dort kann dann durch, insbesondere flächiges, Verschweißen oder Verkleben eine keimdichte Verbindung hergestellt werden.

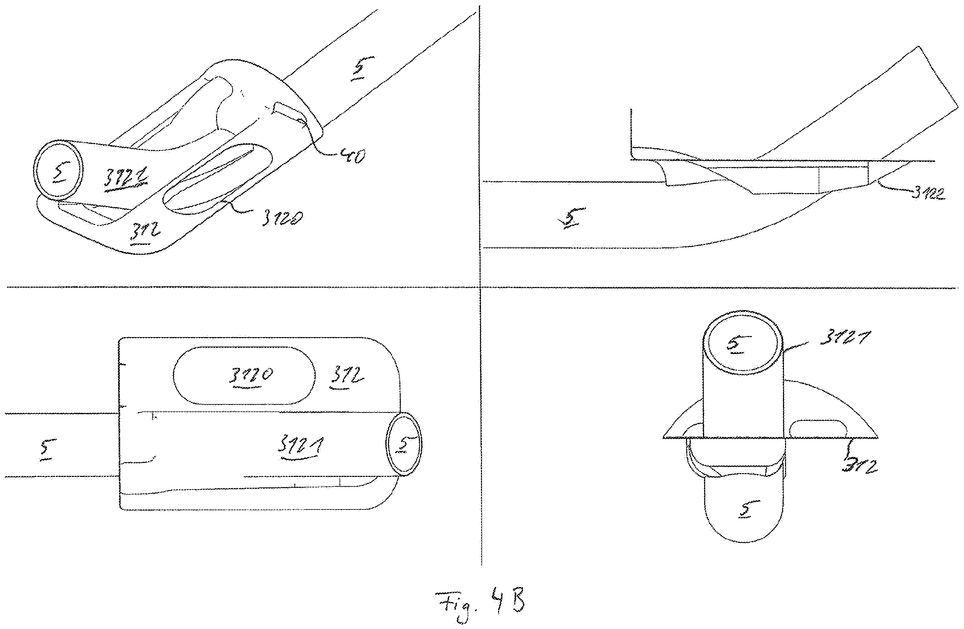

Die **Figuren 2B** - **4C** geben in der Reihenfolge der Nummern die wesentlichen Schritte bei der Herstellung des erfindungsgemäßen Kontaminationsschutzes gemäß vorliegender Erfindung wieder. D.h. nach dem Überziehen der Hülle über den Endoskopschaft (nicht dargestellt) wird zunächst die Kappe aufgesetzt (Figuren 2A,B), mit dem offenen Ende der Hülle keimdicht verbunden, dann wird der Instrumentenschlauch aus dem Arbeitskanal und der Öffnung der Kappe herausgeschoben (Figuren 3A-C), und abschließend die Abdeckung aufgesetzt und keimdicht sowohl mit der Kappe als auch dem Arbeitsschlauch verbunden (Figuren 4A, B). Sofern ein Absaugschlauch auf der Innenseite der Hülle angeordnet ist wird dieser nach dem Aufsetzen der Kappe mit einer als Mündung in dieser vorgesehenen Öffnung angeschlossen (Figur 4C).

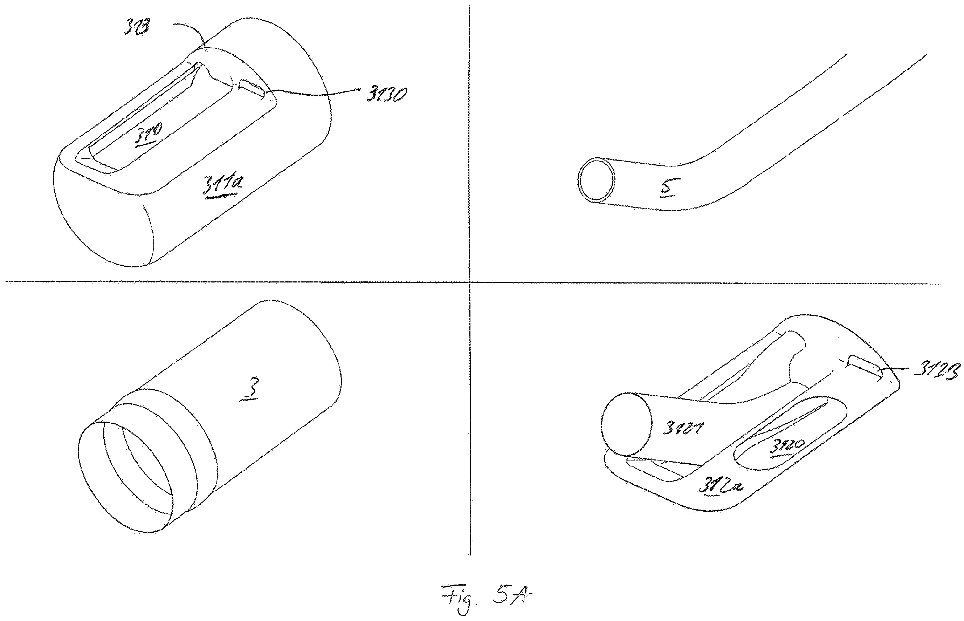

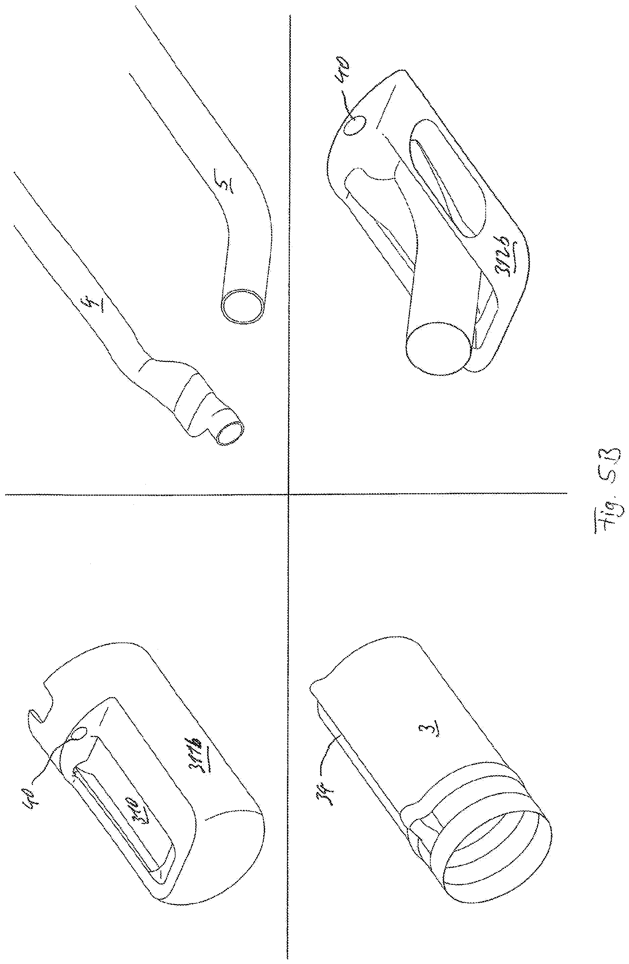

Die **Figuren 5A** und **5B** zeigen in zwei Varianten die Komponenten des erfindungsgemäßen Kontaminationsschutzes bei Verwendung einer Kappe gemäß der ersten Ausführungsform.

In beiden sind in einer ersten Teilfigur links oben die Kappe 311, in einer zweiten Teilfigur links unten ein Abschnitt der den Endoskopschaft bedenkenden Hülle 3, in einer dritten Teilfigur rechts oben die verwendeten Schläuche und in der vierten und letzten Teilfigur rechts unten die keimdichte Abdeckung 312 des offenen Ausschnitts 310 der Kappe 311 gezeigt.

In der Variante nach **Figur 5A** ist in einer senkrecht zur Längsrichtung des Schaftes orientierten Fläche 313 eine längliche Öffnung 3130 vorhanden, welche dem Anschluss der Mündung eines Luft und/oder Wasserschlauches (nicht dargestellt) dient. Entsprechend wird die Kappe 311a dieser Variante mit einer Abdeckung 312a verwendet, welche eine gleichartige, mit der Öffnung 3130 der Kappe 311a fluchtende längliche Öffnung 3123 hat.

In der Variante nach **Figur 5B** ist in der hier verwendeten Kappe 311b anstelle der länglichen Öffnung 3130 der Kappe 311a für einen Luft/Wasserschlauch eine runde Öffnung 40 zum keimdichten Anschluss eines Absaugschlauches 4 vorhanden. Die Hülle 3 hat zur Unterbringung dieses außen am Schaft entlanggeführten Absaugschlauches 4 einen Grat 34.

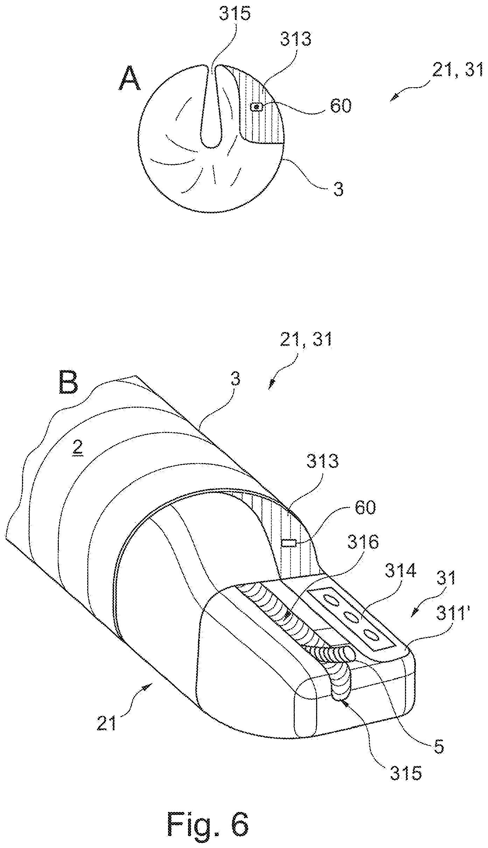

**Figur 6** zeigt in zwei Teilfiguren eine zweite Ausführungsform der Kappe welche das distale Ende der Hülle des erfindungsgemäßen Kontaminationsschutzes bildet. Teilfigur A zeigt eine Vorder- und Teilfigur B eine perspektivische Ansicht.

Dargestellt ist wiederum das distale Ende des Schaftes 2, welches im Bereich oberhalb der Schaftspitze 21 von der Hülle 3 enganliegend bedeckt ist. Das distale Ende der Hülle 3 bildet die Kappe 311' mit dem länglichen Schlitz 315, der dem Arbeitskanalschlauch 5 den Durchtritt durch die ansonsten die Schaftspitze 21 vollständig bedenkende Kappe 311' erlaubt. Der Freiraum zwischen Schaftspitze 21 und den Rändern des Schlitzes 315 wird durch die elastische, faltenbalgartig ausgebildete und so den Bewegungen des Schlauches 5 folgende Folie 316 ausgefüllt und keimdicht verschlossen.

Der oberhalb der Optik und der Beleuchtung liegende Bereich 314 ist als transparentes Fenster ausgebildet um die Benutzung der Optik zu erlauben. In der relativ zur Längsrichtung des Schaftes 2 in einem von Null verschiedenen Winkel ausgerichtete Fläche 313 der des distalen Endes 31 befindet sich die Mündung 60 einer Luft/Wasserinsufflation, welche derart geformt und gerichtet ist, dass sich ein gebündelter Wasserjetstrahl bildet, welcher zunächst das Fenster 314 oberhalb der Optik reinigt und dann als weiterhin gebündelter Strahl zur Benässung einer Untersuchungs- bzw. Operationsstelle eingesetzt werden kann.

### Bezugszeichenliste

- 1: Endoskop
- 2: Endoskopschaft
- 21: Schaftspitze
- 210: Optik, seitwärts gerichtet
- 211: Beleuchtung
- 212: Mündung Luft/Wasserkanal
- 213: Albarranhebel
- 214: Bowdenzug

- 3: Kontaminationsschutz, Hülle
- 30: Befestigungsbereich von 311 an 3
- 31: distales Ende von 3
- 310: Ausschnitt
- 3100: Ausnehmung in 310
- 311, 311': Kappe
- 312: Abdeckung
- 3120: Fenster in 312
- 3121: Kragen
- 3122: Vertiefung
- 3123: Öffnung für Wasser/Luftinsufflation
- 313: geneigte Seitenfläche mit Mündung
- 3130: Mündung Wasser/Luftschlauch in 313
- 314: Fenster in 311'
- 315: Längsschlitz in 311'
- 316: faltenbalgartige Folie zum Verschluss von 315

- 4: Absaugschlauch
- 40: Mündung von 4, Öffnung für 4 in 312

## Patentansprüche

1. Endoskop mit Kontaminationsschutzvorrichtung umfassend
- einen Schaft (2), welcher eine Längsachse definiert und an dessen Spitze (21) eine seitwärts gerichtete Optik (210), eine Beleuchtung (211) und/oder eine Jetdüse (212) angeordnet sind,
- ein an einem der Spitze gegenüberliegenden kranialen Ende des Schafts (2) anschließendes Griffstück,
- eine mindestens den Schaft eng umschließende ein- oder mehrlagige Hülle (3), die an einem die Schaftspitze bedeckenden distalen Ende (31) geschlossen ist, wobei die Hülle (3) zumindest im Bereich der Optik (210) und der Beleuchtung (211) transparent ist,
wobei die Hülle den Schaft (2) und dessen Spitze (21) am distalen Endoskopende keimdicht abschließt, und
das distale Ende (31) der Schafthülle (3) eine Kappe (311) umfasst, welche nach Überstreifen der Hülle (3) über den Schaft (2) aufgesteckt und mit einem offenen Ende (30) der Hülle (3) keimdicht verbunden ist, wobei die Kappe (31)
- die Spitze (21) zumindest auf einer der Optik (210) gegenüberliegenden Rückseite der Spitze (21) und ggf. eines Albarran-hebels (213) keimdicht bedeckt, und
- ein zumindest die Optik (210) und das Beleuchtungsmittel (211) freilassenden Ausschnitt (310) aufweist, der von einer Abdeckung (312) aus einem festen, elastischen, transparenten und keimdichten Material, welche mit der Kappe (311) auf einem ganzen Umfang des Ausschnittes (310) keimdicht verbunden ist, abgedeckt ist,
**dadurch gekennzeichnet, dass**
die Abdeckung (312) durch einen Schlauch (5), der in einem Arbeitskanal des Endoskops (1) platziert ist, perforiert und an der Perforationsstelle fest und keimdicht mit dem Schlauch (5) verbunden ist.

2. Endoskop mit Kontaminationsschutzvorrichtung umfassend
- einen Schaft (2), welcher eine Längsachse definiert und an dessen Spitze (21) eine seitwärts gerichtete Optik (210), eine Beleuchtung (211) und/oder eine Jetdüse (212) angeordnet sind,
- ein an einem der Spitze gegenüberliegenden kranialen Ende des Schafts (2) anschließendes Griffstück,
- eine mindestens den Schaft eng umschließende ein- oder mehrlagige Hülle (3), die an einem die Schaftspitze bedeckenden distalen Ende (31) geschlossen ist, wobei die Hülle (3) zumindest im Bereich der Optik (210) und der Beleuchtung (211) transparent ist,
wobei die Hülle den Schaft (2) und dessen Spitze (21) am distalen Endoskopende keimdicht abschließt, und
das distale Ende (31) der Schafthülle (3) eine Kappe (311) umfasst, welche nach Überstreifen der Hülle (3) über den Schaft (2) aufgesteckt und mit einem offenen Ende (30) der Hülle (3) keimdicht verbunden ist, wobei die Kappe (31) die Spitze (21) vollständig keimdicht bedeckt und zumindest im Bereich der Optik (210) und der Beleuchtung (211) ein transparentes Fenster (314) aufweist,
**dadurch gekennzeichnet, dass**
die Kappe (311) über einen in Längsrichtung des Schaftes (2) verlaufenden Schlitz (315) verfügt, durch den ein Schlauch (5), der in einem Arbeitskanal des Endoskops (1) platziert ist, die Kappe (311) durchdringt, wobei ein offener Bereich zwischen dem Schlauch (5) und Rändern des Schlitzes (315) mittels eines faltenbalgartigen und/oder elastischer Folienbereich (316) keimdicht ausgefüllt ist.

3. Endoskop nach Anspruch 1 oder 2, **gekennzeichnet durch** eine in der Spitze 21 befindliche Mechanik, welche insbesondere einen Albarranhebel (213) umfasst.

4. Endoskop nach einem der vorhergehenden Ansprüche, **gekennzeichnet durch** einen Schlauch (4) für eine Luft- und/oder Wasserinsufflation, insbesondere einen kombinierten Luft/Wasserschlauch oder zwei getrennte Schläuche für Luft und Wasser, der
- in der Nähe des distalen Endes (31) der Hülle (3) offen, und
- dort keimdicht mit der Hülle (3) verbunden ist, und
- sich innerhalb der Hülle (3) befindet.

5. Endoskop nach Anspruch 4, **dadurch gekennzeichnet, dass** der Schlauch (4) am kranialen Ende, außerhalb der Hülle mit einem integriertem Ventil zur Luft/Wasserinsufflation verbunden ist, wobei das Ventil in eine Ventilaufnahme des Endoskops (1) als Halterung steckbar gestaltet ist.

6. Endoskop nach einem der Ansprüche 1 - 5, **dadurch gekennzeichnet, dass** die die Hülle (3) doppellagig ist.

7. Endoskop nach einem der Ansprüche 4 - 6, **dadurch gekennzeichnet, dass** der Luft und/oder Wasserschlauch (4) mit einem distalen Ende in einer zur Längsachse einen Winkel von zwischen 30 bis 90 Grad einnehmenden, insbesondere senkrechten Fläche (313) der Kappe (311) mündet, wobei die Mündung (40) eine Geometrie und Ausrichtung aufweist, durch die ein Wasserstrahl auf die Optik (210) und/oder die Beleuchtung (211) geleitet werden kann.

8. Endoskop nach Anspruch 7, **dadurch gekennzeichnet, dass** die Mündung (40) eine Geometrie und Ausrichtung aufweist, durch welche ein aus der Mündung (40) austretender Wasserstrahl über die Optik (210) und/oder die Beleuchtung hinaus einen gebündelten Strahl bildet.

## Claims

1. Endoscope with a contamination protection device comprising
- a shaft (2) which defines a longitudinal axis and which has, positioned at its tip (21), a sideways directed optics (210), a lighting (211) and/or a jet nozzle (212),
- a grip piece adjacent to a cranial end of the shaft (2) opposite to the tip,
- a single- or multi-layered sheath (3) which tightly encloses at least the shaft and which is closed at a distal end (31) that covers the shaft tip, wherein the sheath (3) is transparent at least in the region of the optics (210) and the lighting (211),
wherein the sheath closes the shaft (2) and its tip (21) at the distal end of the endoscope in a germ-tight manner, and
the distal end (31) of the sheath (3) comprises a cap (311), which is plugged on after the sheath (3) has been slipped over the shaft (2) and is connected in a germ-tight manner with an open end (30) of the sheath (3), wherein the cap (31)
- sheaths the tip (21) in a germ-tight manner at least on a rear side of the tip (21) which is opposite to the optics (210) and, if applicable, an Albarran-lever (213), and
- has a cutout (310) which leaves open at least the optics (210) and the lighting means (211) and which is covered by a coverage (312) made of a firm, elastic, transparent and germ-proof material that is connected to the cap (311) in a germ-tight manner along an entire circumference of the cutout (310),
**characterized in that**
the coverage (312) is penetrated by a tube (5) that is placed inside a working channel of the endoskope (1) and is connected to the tube (5) at the penetration location in a solid and germ-tight manner.

2. Endoscope with a contamination protection device comprising
- a shaft (2) which defines a longitudinal axis and which has, positioned at its tip (21), a sideways directed optics (210), a lighting (211) and/or a jet nozzle (212),
- a grip piece adjacent to a cranial end of the shaft (2) opposite to the tip,
- a single- or multi-layered sheath (3) which tightly encloses at least the shaft and which is closed at a distal end (31) that sheaths the shaft tip, wherein the sheath (3) is transparent at least in the region of the optics (210) and the lighting (211),
wherein the sheath closes the shaft (2) and its tip (21) at the distal end of the endoscope in a germ-tight manner, and
the distal end (31) of the shaft sheath (3) comprises a cap (311), which is plugged on after the sheath (3) has been slipped over the shaft (2) and is connected in a germ-tight manner with an open end (30) of the sheath (3), wherein the cap (31) completely covers the tip (21) in a germ-tight manner and has a transparent window (314) at least in the region of the optics (210) and the lighting (211),
**characterized in that**
the cap (311) has a slit (315) running in the longitudinal direction of the shaft (2), through which a tube (5) that is placed in a working channel of the endoscope (1), pierces the cap (311), wherein an open area between the tube (5) and edges of the slit (315) is filled in a germ-tight manner by a bellows-like and/or elastic sheet region (316).

3. Endoscope according to claim 1 or 2, **characterized by** a mechanism located inside the tip (21), which comprises in particular an Albarran-lever (213).

4. Endoscope according to one of the preceding claims, **characterized by** a tube (4) for an air and/or water insufflation, in particular a combined air/water tube or two separate tubes for air and water, which is/are
- open in the vicinity of the distal end (31) of the sheath (3), and
- there connected to the sheath (3) in a germ-tight manner, and
- located inside the sheath (3).

5. Endoscope according to claim 4, **characterized in that** the tube (4) is, at a cranial end outside the sheath, connected to an integrated valve for air/water insufflation, wherein the valve is fashioned to be pluggable into a valve retainer of the endoscope (1) as a fixture.

6. Endoscope according to one of the claims 1 - 5, **characterized in that** the sheath (3) is two layered.

7. Endoscope according to one of the claims 4-6, **characterized in that** the air and/or water tube (4) ends in an outlet (40) in a face (313) of the cap (311) which has an angle of between 30 and 90 degrees with, in particular is perpendicular to, the longitudinal axis, wherein the outlet (40) has a geometry and alignment by which a water jet can be directed onto the optics (210) and/or the lighting (211).

8. Endoscope according to claim 7, **characterized in that** the outlet (40) has a geometry and alignment by which a water jet issuing from the outlet (40) forms a focused jet even beyond the optics (210) and the lighting.

## Revendications

1. Endoscope avec dispositif anti-contamination comprenant
- un arbre (2) qui définit un axe longitudinal et à la pointe (21) duquel sont disposés une optique latérale (210), un éclairage (211) et/ou une buse à jet (212),
- une poignée fixée à une extrémité crânienne de l'arbre (2) opposée à la pointe,
- un manchon (3) monocouche ou multicouche qui enferme étroitement au moins l'arbre et est fermé à une extrémité distale (31) qui recouvre la pointe du arbre, le manchon (3) étant transparents au moins dans la zone de l'optique (210) et l'éclairage (211),
dans lequel le manchon scelle l'arbre (2) et sa pointe (21) de manière étanche aux germes à l'extrémité distale de l'endoscope,
et
l'extrémité distale (31) du manchon du arbre (3) comprend un capuchon (311) qui, après que le manchon (3) a été enfilé sur l'arbre (2), est placé dessus et est lié avec une extrémité ouverte (30) du manchon (3) a manière étanche aux germes, le capuchon (311)
- recouvre la pointe (21) de manière étanche aux germes au moins sur un côté arrière de la pointe (21) et, eventuellement, d'un levier élévateur (213) opposé à l'optique (210), et
- comporte un évidement (310) qui laisse libre au moins l'optique (210) et les moyens d'éclairage (211) et qui est recouverte d'un couvercle (312) réalisé en un matériau solide, élastique, transparent et anti-germe, qui est relié avec le capuchon (311) sur toute la circonférence de l'évidement (310) de manière étanche aux germes,
**caractérisé en ce que**
le couvercle (312) est perforé par un tuyau (5) qui est placé dans un canal opérateur de l'endoscope (1) et est relié solidement et étanchement au tuyau (5) au point de perforation.

2. Endoscope avec dispositif anti-contamination comprenant
- un arbre (2) qui définit un axe longitudinal et à la pointe (21) duquel sont disposés une optique latérale (210), un éclairage (211) et/ou une buse à jet (212),
- une poignée fixée à une extrémité crânienne du arbre (2) opposée à la pointe,
- un manchon (3) monocouche ou multicouche qui enferme étroitement au moins l'arbre et est fermé à une extrémité distale (31) qui recouvre la pointe du arbre, le manchon (3) étant transparent au moins dans la zone de l'optique (210) et l'éclairage (211),
dans lequel le manchon scelle l'arbre (2) et sa pointe (21) de manière étanche aux germes à l'extrémité distale de l'endoscope, et
l'extrémité distale (31) du manchon du arbre (3) comprend un capuchon (311) qui, après que le manchon (3) a été enfilé sur l'arbre (2), est placé dessus et est lié avec une extrémité ouverte (30) du manchon (3) a manière étanche aux germes, le capuchon (311) recouvrant complètement la pointe (21) de manière anti-germe et présentant une fenêtre transparente (314) au moins dans la zone de l'optique (210) et l'éclairage (211),
**caractérisé en ce que**
le capuchon (311) présente une fente (315) s'étendant dans la direction longitudinale de l'arbre (2) à travers laquelle un tuyau (5) placé dans un canal de travail de l'endoscope (1) pénètre dans le capuchon (311), la zone ouverte située entre le tuyau (5) et les bords de la fente (315) étant remplie d'un joint étanche aux germes au moyen d'une zone de film en forme de soufflet et/ou élastique (316).

3. Endoscope selon la revendication 1 ou 2, **caractérisé par** un mécanisme situé dans la pointe (21), qui comprend notamment un levier élévateur (213).

4. Endoscope selon l'une des revendications précédentes, **caractérisé par** un tuyau (4) d'insufflation d'air et/ou d'eau, notamment un tuyau combiné air/eau ou deux tuyaux séparés pour l'air et l'eau
- étant ouvert à proximité de l'extrémité distale (31) de la manchon (3), et
- ayant une liaison étanche aux germes avec la manchon (3),
et
- étant situé à l'intérieur du manchon (3).

5. Endoscope selon la revendication 4, **caractérisé en ce que** le tuyeaux (4) est relié à l'extrémité crânienne, à l'extérieur du manchon, à une valve intégrée d'insufflation air/eau, la valve pouvant s'enficher comme support dans un logement de valve du endoscope (1).

6. Endoscope selon l'une des revendications 1 à 5, **caractérisé en ce que** la manchon (3) est à double couches.

7. Endoscope selon l'une des revendications 4 à 6, **caractérisé en ce que** le tuyeau d'air et/ou d'eau (4) se termine par une bouche (40) dans une face (313) du capuchon (311) qui présente un angle compris entre 30 et 90 degrés avec, en particulier est perpendiculaire à, l'axe longitudinal, dans lequel la bouche (40) a une géométrie et un alignement par lesquels un jet d'eau peut être dirigé sur l'optique (210) et/ou l'éclairage (211).

8. Endoscope selon la revendication 7, **caractérisé en ce que** la bouche (40) a une géométrie et un alignement par lesquels un jet d'eau sortant de la bouche (40) forme un faisceau focalisé au-delà de l'optique (210) et/ou de l'éclairage.
